# EUROPEAN PATENT APPLICATION

(11) **EP 0 355 065 A1**
(43) Date of publication of application: **21.02.1990**
(21) Application number: 89308393.1
(22) Date of filing: 18.08.1989
(51) Int. Cl.: C07K 5/02, A61K 37/64

(54) **Renin inhibitory peptides containing suleptanic acid or derivatives thereof**

(30) Priority: 19.08.1988 US 234413; 20.07.1989 WO PCT/US89/03093
(71) Applicant: THE UPJOHN COMPANY, Kalamazoo, Michigan 49001 (US)
(72) Inventor: Thaisrivongs, Suvit, Portage Michigan 49002 (US)
(74) Representative: Perry, Robert Edward

(57) **Abstract**

The present invention provides novel renin-inhibiting peptides having a non-cleavable transition state insert corresponding to the 10,11- position of the renin substrate (angiotensinogen) and having a suleptanic acid moiety or derivatives thereof of the formula L₁ at the N-terminus of the peptide. Such inhibitors are useful for the diagnosis and control of renin-dependent hypertension, congestive heart failure, renin dependent hyperaldosterism, other renin dependent cardiovascular disorders and ocular disorders.

Y₁0₃S-(CH₂)ₙ-N(R₁)- 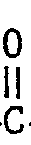 -(CH₂)_{q}- 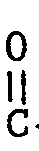 - L₁

## Description

### BACKGROUND OF THE INVENTION

The present invention provides novel compounds. More particularly, the present invention provides novel renin-inhibiting peptide analogs. Most particularly, the present invention provides renin-inhibitory peptides containing a non-cleavable transition state insert corresponding to the 10,11-position of the renin substrate (angiotensinogen) and containing a suleptanic acid moiety H0₃S(CH₂)₂-N(CH₃)C0(CH₂)₆C0₂H or derivatives thereof at the N-terminus of the peptide. The renin inhibitors provided herein are useful for the diagnosis and control of renin-dependent hypertension, congestive heart failure, renin dependent hyperaldosterism, and other renin dependent cardiovascular disorders.

Renin is an endopeptidase which specifically cleaves a particular peptide bond of its substrate (angiotensinogen), of which the N-terminal sequence in equine substrate is for example: 
Renin
↓
Asp-Arg-Val-Tyr-Ile-His-Pro-Phe-His-Leu-Leu-Val-Tyr-Ser-     IA
1   2   3   4   5   6   7   8   9   10  11  12  13  14
 as found by L.T. Skeggs et al., J. Exper. Med. 106, 439 (1957). Human renin substrate has a different sequence as recently discovered by D.A. Tewkesbury et al., Biochem. Biophys. Res. Comm. 99, 1311 (1981). It may be represented as follows: 
Renin
  ↓
  -Val-Ile-His-
   11  12  13                    IB
 and having the sequence to the left of the arrow (↓) being as designated in formula IA above.

Renin cleaves angiotensinogen to produce angiotensin I, which is converted to the potent pressor angiotensin II. A number of angiotensin I converting enzyme inhibitors are known to be useful in the treatment of hypertension. Inhibitors of renin are also useful in the treatment of hypertension.

A number of renin-inhibitory peptides have been disclosed. Thus, U.S. patent 4,424,207; European published applications 45,665; 104,041; and 156,322; and U.S. patent application, Serial No. 825,250, filed 3 February 1986; disclose certain peptides with the dipeptide at the 10,11-position containing an isostere bond. A number of statine derivatives stated to be renin inhibitors have been disclosed, see, e.g., European published applications 77,028; 81,783; 114,993; 156,319; and 156,321; and U.S. patents 4,478,826; 4,470,971; 4,479,941; and 4,485,099. Terminal disulfide cycles have also been disclosed in renin inhibiting peptides; see, e.g., U.S. patents 4,477,440 and 4,477,441. Aromatic and aliphatic amino acid residues at the 10,11 position of the renin substrate are disclosed in U.S. patents 4,478,827 and 4,455,303. C-terminal amide cycles are disclosed in U.S. patent 4,485,099 and European published applications 156,320 and 156,318. Certain tetrapeptides are disclosed in European publications 111,266 and 77,027. Further, European published application No. 118,223 discloses certain renin inhibiting peptide analogs where the 10-11 peptide link is replaced by a one to four atom carbon or carbon-nitrogen link. Additionally, Holladay et al., in "Synthesis of Hydroxyethylene and Ketomethylene Dipeptide Isosteres", Tetrahedron Letters, Vol. 24, No. 41, pp. 4401-4404, 1983 disclose various intermediates in a process to prepare stereo-directed "ketomethylene" and "hydroxyethylene" dipeptide isosteric functional groups disclosed in the above noted U.S. Patent No. 4,424,207.

Additionally, published European Applications 45,161 and 53,017 disclose amide derivatives useful as inhibitors of angiotensin converting enzymes.

Certain dipeptide and tripeptides are disclosed in U.S. patents 4,514,332; 4,510,085; and 4,548,926 as well as in European published applications 128,762; 152,255; and 181,110. Pepstatin derived renin inhibitors have been disclosed in U.S. patent 4,481,192. Retroinverso bond modifications at positions 10-11 have been disclosed in U.S. patent 4,560,505 and in European published applications 127,234 and 127,235. Derivatives of isosteric bond replacements at positions 10-11 have been disclosed in European published applications 143,746 and 144,209; and U.S. patent application, Serial No. 833,993, filed 27 February 1986. Isosteric bond modifications at positions 11-12 and 12-13 have been disclosed in European published application 179,352. Certain peptides containing 2-substituted statine analogues have been disclosed in European published application 157,409. Certain peptides containing 3-aminodeoxystatine have been disclosed in European published application 161,588. Certain peptides containing 1-amino-2-hydroxybutane derivatives at positions 10-11 have been disclosed in European published application 172,346. Certain peptides containing 1-amino-2-hydroxypropane derivatives at positions 10-11 have been disclosed in European published application 172,347. Certain peptides containing N-terminal amide cycles have been disclosed in U.S. patent application, Serial No. 844,716, filed 27 March 1986. Certain peptides containing dihalostatine have been disclosed in PCT application, Serial No. 000,713, filed 7 April 1986.

European published applications 156,322; 114,993; and 118,223; and U.S. patent application, Serial No. 798,459, filed 15 November 1985; U.S. patent application, Serial No. 825,250, filed 3 February 1986; U.S. patent application, Serial No. 833,993, filed 27 February 1986; and U.S. patent application, Serial No. 844,716, filed 27 March 1986, disclose hydroxamic acids or esters at the C-terminus.

### INFORMATION DISCLOSURE

Several THAM amides of Leu-Val alcohol-based renin inhibitors are disclosed in Australian Patent Au-A-35804/84. (The U.S. equivalent is U.S. Patent 4,613,676 and the Basic Patent is 0-143-746 (Europe).)

U.S. Patent Application, Serial No. 151,129, filed 1 February 1988, discloses renin inhibitory peptides having a variety of polar end groups at the N-terminus and/or the C-terminus.

### SUMMARY OF THE INVENTION

The present invention particularly provides:

A renin inhibitory peptide having a non-cleavable transition state insert corresponding to th 10,11-position of a renin substrate (angiotensinogen) and having a moiety of the formula L₁ at the N-terminus;
wherein Y₁ is
(a) hydrogen,
(b) alkali metals,
(c) alkali earth metals, or
(d) pharmaceutically acceptable base addition salts thereof;
wherein R₁ is
(a) hydrogen, or
(b) C₁-C₅alkyl;
wherein n is 1 to 5, inclusive; and
wherein q is 1 to 8, inclusive;
in a renin inhibitory peptide having a non-cleavable transition state insert corresponding to the 10,11-position of a renin substrate (angiotensinogen), the improvement which comprises inclusion in the renin inhibitory peptide of a moiety of the formula L₁ at the N-terminus;
wherein Y₁ is
(a) hydrogen,
(b) alkali metals,
(c) alkali earth metals, or
(d) pharmaceutically acceptable base addition salts thereof;
wherein R₁ is
(a) hydrogen, or
(b) C₁-C₅alkyl;
wherein n is 1 to 5, inclusive; and
wherein q is 1 to 8, inclusive;
the renin inhibitory peptide of the formula I
wherein A₆ is a monovalent moiety of the formula L₁;
wherein B₇ is absent or a divalent moiety of the formula L₂;
wherein D₈ is a divalent moiety of the formula L₃ or L₄;
wherein E₉ is a divalent moiety of the formula L₄ or L₅;
wherein each occurrence of the moiety of the formula L₄ can be the same or different;
wherein V₁ is
(a) -0-, or
(b) -N(R₁)-;
wherein X₁ is
(a) -CH(0H)-CH(0H)-CH₂-P₁,
(b) -L₁₀-C(R₁)(R₄)-C(0)-F₁-Z₁, or
(c) -J₁-C(K₁)(K₂)-C(0)-F₁-Z₁;
wherein P₁ is
(a) -N₃,
(b) -CN,
(c) C₁-C₆alkyl,
(d) C₁-C₆cycloalkyl,
(e) aryl, or
(f) Het;
wherein L₁₀ is a divalent moiety of the formula
(a) -CH(0H)-,
(b) -CH(NH₂)-,
(c) -C(0)-,
(d) -CH(0H)-C(K₁)(K₂)-,
(e) -C(0)-C(K₁)(K₂)-,
(f) -CH(0H)-CH(0H)-,
(g) -CH(0H)-CH₂-,
(h) -CH(NH₂)-CH₂-,
(i) -C(0)-CH₂-,
(j) -CH₂-NH-,
(k) -CH₂-0-, or
(l) -P₁(0)(G₁)-I₁-;
wherein F₁ is absent or a divalent moiety of the formula L₄;
wherein G₁ is
(a) -0H, or
(b) -NH₂;
wherein I₁ is
(a) -0-,
(b) -NH-, or
(c) -CH₂;
wherein J₁ is
(a) -CH(0H)-,
(b) -CH(NH₂)-, or
(c) -C(0)-;
wherein K₁ and K₂ are the same or different and are
(a) H,
(b) F, or
(c) C1;
wherein Q₁ is
(a) -CH₂-,
(b) -CH(0H)-,
(c) -0-, or
(d) -S-;
wherein M₁ is
(a) -C(0)-, or
(b) -CH₂-;
wherein Y₁ is
(a) hydrogen,
(b) alkali metals,
(c) alkali earth metals, or
(d) pharmaceutically acceptable base addition salts thereof;
wherein Z₁ is
(a) -0-R₅, or
(b) -N(R₁)R₅;
wherein R₁ is
(a) hydrogen, or
(b) C₁-C₅alkyl;
wherein R₂ is
(a) hydrogen,
(b) C₁-C₅alkyl,
(c) C₃-C₇cycloalkyl,
(d) aryl,
(e) Het,
(f) -(CH₂)ₚ-0H, or
(g) -(CH₂ₚ-NH₂;
wherein R₃ is
(a) C₁-C₅alkyl,
(b) C₃-C₇cycloalkyl,
(c) aryl, or
(d) Het;
wherein R₄ is
(a) hydrogen,
(b) C₁-C₈alkyl,
(c) -(CH₂)ₚ-aryl,
(d) -(CH₂)ₚ-Het,
(e) -(CH₂)ₚ-C0₂H,
(f) -(CH₂)ₚ-NH₂,
(g) -(CH₂)ₚ-CH(NH₂)(C0₂H),
(h) C₃-C₇cycloalkyl, or
(i) 1- or 2-adamantyl;
wherein R₅ is
(a) hydrogen,
(b) C₁-C₁₀alkyl,
(c) aryl,
(d) Het,
(e) -(CH₂)ₚ-(C₃-C₇cycloalkyl),
(f) -(CH₂)ₚ-CH(NH₂)(C0₂H), or
(g) -(CH₂)ₙ-R₆;
wherein R₆ is
(a) aryl,
(b) Het,
(c) hydroxy,
(d) amino,
(e) C₁-C₅alkyl substituted by 1 to 3 -0H groups,
(f) -C0₂H,
(g) guanidinyl,
(h) -S0₃H, or
(i) -S0₂NH₂;
wherein R₇ is
(a) hydrogen, or
(b) C₁-C₅alkyl;
wherein R₈ is
(a) hydrogen, or
(b) C₁-C₅alkyl;
wherein R₉ is
(a) hydrogen, or
(b) C₁-C₅alkyl;
wherein R₁₀ is
(a) hydrogen,
(b) C₁-C₈alkyl,
(c) -(CH₂)ₚ-aryl,
(d) -(CH₂)ₚ-Het,
(e) -(CH₂)ₚ-C0₂H,
(f) -(CH₂)ₚ-NH₂,
(g) -(CH₂)ₚ-CH(NH₂)(C0₂H),
(h) C₃-C₇cycloalkyl, or
(i) 1- or 2-adamantyl;
wherein R₁₁ is
(a) hydrogen, or
(b)C₁-C₅alkyl;
wherein m is 1 or 2;
wherein n is 1 to 5, inclusive;
wherein p is 0 to 5, inclusive;
wherein q is 1 to 8, inclusive;
wherein r is 1 or 2;
wherein aryl is phenyl or naphthyl, optionally substituted by zero to three of the following;
(a) C₁-C₅alkyl,
(b) hydroxy,
(c) hydroxy (C₁-C₅alkyl),
(d) halogen,
(e) amino,
(f) amino (C₁-C₅alkyl),
(g) -CH0,
(h) -C0₂H,
(i) -C0₂-(C₁-C₅alkyl),
(j) -C0NH₂,
(k) -CONH-(C₁-C₅alkyl),
(l) nitro,
(m) mercapto,
(n) mercapto (C₁-C₅alkyl),
(o) -S0₃H,
(p)-S0₂NH₂,
(q) -CN, or
(r) -0-C₁-C₅alkyl;
wherein Het is a 5 or 6-membered saturated or unsaturated ring containing from one to three heteroatoms selected from the group consisting of nitrogen, oxygen and sulfur; and including any bicyclic group in which any of the above heterocyclic rings is fused to a benzene ring or another heterocycle; and if chemically feasible, the nitrogen and sulfur atoms may be in the oxidized forms; and optionally substituted by zero to three of the following;
(a) C₁-C₅alkyl,
(b) hydroxy,
(c) hydroxy (C₁-C₅alkyl),
(d) halogen,
(e) amino,
(f) amino (C₁-C₅alkyl),
(g) -CH0,
(h) -C0₂H,
(i) -C0₂-(C₁-C₅alkyl),
(j) -C0NH₂,
(k) -CONH-(C₁-C₅alkyl),
(l) nitro,
(m) mercapto,
(n) mercapto (C₁-C₅alkyl),
(o) -S0₃H,
(p) -S0₂NH₂,
(q) -CN, or
(r) -0-C₁-C₅alkyl;
or a carboxy-, amino- or other reactive group protected form thereof;
or a pharmaceutically acceptable acid or base addition salts thereof.

By "renin inhibitory peptide" is meant a compound capable of inhibiting the renin enzyme in mammalian metabolism and having three or more amino acid residues linked by peptidic or pseudo-peptidic bonds.

By a "non-cleavable transition state insert" is meant a transition state insert which is not cleavable by a hydrolytic enzyme in mammalian metabolism. A variety of such transition state inserts, corresponding to the 10,11-position of the renin substrate, are known in the art, including those disclosed in the following references, which are hereby incorporated by reference:

U.S. Patent 4,424,207 (Szelke); European Patent 104041A (Szelke); European Patent Application 144,290A (Ciba Geigy AG); European Patent 0,156,322 (Merck); European Patent 161-588A (Merck); European Patent 0,172,347 (Abbott); European Patent 172-346-A(Abbott); European Patent 156-318 (Merck); European Patent 157-409 (Merck); European Patent 152-255 (Sankyo); and U.S. Patent 4,548,926 (Sankyo); and

U.S. patent application, Serial No. 904,149, filed 5 September 1986; U.S. patent application, Serial No. 844,716, filed 27 March 1986; PCT application, Serial No. 000,713, filed 7 April 1986; U.S. patent application, Serial No. 945,340, filed 22 December 1986; and U.S. patent application, Serial No. 825,250, filed 3 February 1986; and

A. Spaltenstein, P. Carpino, F. Miyake and P.B. Hyskins, Tetrahedron Letters, 27:2095 (1986); D.H. Rich and M.S. Bernatowicz, J. Med. Chem., 25:791 (1982); Roger, J. Med. Chem., 28:1062 (1985); D.M. Glick et al., Biochemistry,21:3746 (1982); D.H.Rich, Biochemistry, 24:3165 (1985); R.L. Johnson, J. Med. Chem., 25:605 (1982); R.L. Johnson and K. Verschover, J. Med. Chem., 26:1457 (1983); R.L. Johnson, J. Med. Chem., 27:1351 (1984); P.A. Bartlett et al., J. Am. Chem. Soc., 106:4282 (1984); and Peptides: Synthesis, Structure and Function (V.J. Hruby; D.H. Rich, eds.) Proc. 8th American Peptide Sym., Pierce Chemical Company, Rockford, Ill., pp. 511-20; 587-590 (1983).

The renin inhibitory peptides of the present invention are represented by formula I. In formula I, the non-cleavable transition state insert, corresponding to the 10,11-position of the renin substrate, begins at -NHCH(CH₂R₂)X₁, with the variables as defined above.

The present invention provides novel renin inhibitory peptides derived from known renin inhibitory peptides in which the N-terminal tert-butyloxycarbonyl protecting group has been replaced by suleptanic acid or derivatives thereof. The resulting compounds have increased water solubility and retain high renin inhibitory activity. They are also useful to prepare other active renin inhibitory peptides with high water solubility.

As is apparent to those of ordinary skill in the art, the renin inhibitory peptides of the present invention can occur in several isomeric forms, depending on the configuration around the asymmetric carbon atoms. All such isomeric forms are included within the scope of the present invention. Preferably, the stereochemistry of the amino acids corresponds to that of the naturally-occurring amino acids.

Renin inhibitory peptides commonly have protecting groups at the C-terminus. These protecting groups are known in the polypeptide art. Examples of these protecting groups are given below. Any of these protecting groups are suitable for the renin inhibitory peptides of the present invention.

Examples of pharmaceutically acceptable acid addition salts include: acetate, adipate, alginate, aspartate, benzoate, ben zenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, 3-phenylpropionate, picrate, pivalate, propionate, succinate, tartrate, thiocyanate, tosylate, and undecanoate.

The carbon atom content of various hydrocarbon-containing moieties is indicated by a prefix designating the minimum and maximum number of carbon atoms in the moiety, i.e., the prefix (Cᵢ-Cⱼ) indicates a moiety of the integer "i" to the integer "j" carbon atoms, inclusive.Thus (C₁-C₄)alkyl refers to alkyl of one to 4 carbon atoms, inclusive, or methyl, ethyl, propyl, butyl, and isomeric forms thereof. C₄-C₇cyclic amino indicates a monocyclic group containing one nitrogen and 4 to 7 carbon atoms.

Examples of (C₃-C₁₀)cycloalkyl, which include alkyl-substituted cycloalkyl containing a total of up to 10 total carbon atoms, are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, cyclopentyl, 2,2-dimethylcyclopentyl, cyclohexyl, cycloheptyl, cyclooctyl, cyclononyl, cyclodecyl and isomeric forms thereof.

Examples of aryl include phenyl, naphthyl (o-, m-, or p-)tolyl, (o-, m-, or p-)ethylphenyl, 2-ethyl-tolyl, 4-ethyl-o-tolyl, 5-ethyl-m-tolyl, (o-, m-, or p-)propylphenyl, 2-propyl-(o-, m-, or p-)tolyl, 4-isopropyl-2,6-xylyl, 3-propyl-4-ethylphenyl, (2,3,4-2,3,6- or 2,4,5-)trimethylphenyl, (o-, m-, or p-(fluorophenyl, (o-, m-, or p-trifluoromethyl)phenyl, 4-fluoro-2,5-xylyl, (2,4-, 2,5-, 2,6-, 3,4-, or 3,5-)difluorophenyl, (o-, m-, or p-)chlorophenyl, 2-chloro-p-tolyl, (3-, 4-, 5- or 6-)chloro-o-tolyl, 4-chloro-2-propylphenyl, 2-isopropyl-4-chlorophenyl, 4-chloro-3-fluorophenyl, (3- or 4-)chloro-2-fluorophenyl, (o-, m-, or p-)trifluoro-methylphenyl, (o-, m-, or p-)ethoxyphenyl, (4- or 5-)chloro-2-methoxy-phenyl, and 2,4-dichloro(5- or 6-)methylphenyl, and the like.

Examples of -Het include: 2-, 3-, or 4-pyridyl, imidazolyl, indolyl, Nⁱⁿ-formyl-indolyl, Nⁱⁿ-C₁-C₅alkyl-C(0)-indolyl, 1,2,4-triazolyl, 2-, 4-, or 5-pyrimidinyl, 2- or 3-thienyl, piperidinyl, pyrryl, pyrrolinyl, pyrrolidinyl, pyrazolyl, pyrazolinyl, pyrazolidinyl, imidazolinyl, imidazolidinyl, pyrazinyl, piperazinyl, pyridazinyl, oxazolyl, oxazolidinyl, isoxazolyl, isoxazolidinyl, morpholinyl, thiazolyl, thiazolidinyl, isothiazolyl, isothiazolidinyl, quinolinyl, isoquinolinyl, benzimidazolyl, benzothiazolyl, benzoxazolyl, furyl, thienyl, and benzothienyl. Each of these moieties may be substituted as noted above.

As would be generally recognized by those skilled in the art of organic chemistry, a heterocycle as defined herein for -Het would not be bonded through oxygen or sulfur or through nitrogen which is within a ring and part of a double bond.

Halo is halogen (fluoro, chloro, bromo, or iodo) or trifluoromethyl.

Examples of pharmaceutically acceptable cations include: pharmacologically acceptable metal cations, ammonium, amine cations, or quaternary ammonium cations. Especially preferred metal cations are those derived from the alkali metals, e.g., lithium, sodium, and potassium, and fromthe alkaline earth metals, e.g., magnesium and calcium, although cationic forms of other metals, e.g., aluminum, zinc, and iron are also within the scope of this invention. Pharmacologically acceptable amine cations are those derived from primary, secondary, or tertiary amines.

The novel peptides herein contain both natural and synthetic amino acid residues. These residues are depicted using standard amino acid abbreviations (see, e.g., IUPAC-IUB Joint Commission on Biochemical Nomenclature (JCBN), "Nomenclature and Symbolism for Amino Acids and Peptides," Eur. J. Biochem. 138:9-37 (1984) unless otherwise indicated.

The renin inhibitors of this invention are useful for treating any medical condition for which it is beneficial to reduce the levels of active circulating renin. Examples of such conditions include renin-dependent hypertension, hypertension, hypertension under treatment with another antihypertensive and/or a diuretic agent, congestive heart failure, renin-dependent hyperaldosterism, angina, post-myocardial infarction, other renin-dependent cardiovascular disorders and ocular disorders. The renin-angiotension system may play a role in maintenance of intracellular homeostasis: see Clinical and Experimental Hypertension, 86, 1739-1742 (1984) at page 1740 under Discussion.

The compounds of the present invention are preferably orally administered to humans to effect renin inhibition for the purpose of favorably affecting blood pressure. For this purpose, the compounds are administered from 0.1 mg to 100 mg per kg per dose, administered from 1 to 4 times daily. Equivalent dosages for other routes of administration are also employed. For example, renin-associated hypertension and hyperaldosteronism are effectively treated by the administration of from 0.5 to 50 milligrams of the compound per kilogram of body weight per day.

The exact dose depends on the age, weight, and condition of the patient and on the frequency and route of administration. Such variations are within the skill of the practitioner or can readily be determined.

The compounds of the present invention may be in the form of pharmaceutically acceptable salts both those which can be produced from the free bases by methods well known in the art and those with which acids have pharmacologically acceptable conjugate bases.

Conventional forms and means for administering renin-inhibiting compounds may be employed and are described, e.g., in U.S. Patent No. 4,424,207 which is incorporated by reference herein. Likewise, the amounts disclosed in the U.S. patent No. 4,424,207 are examples applicable to the compounds of the present invention.

The compounds of the present invention are preferably orally administered in the form of pharmacologically acceptable acid addition salts. Preferred pharmacologically acceptable salts for oral administration include the citrate and aspartate salts, although any pharmacologically acceptable salt is useful in this invention, including those listed above. These salts may be in hydrated or solvated form.

For these purposes the compounds of the present invention may be administered topically, parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerids. In addition, fatty acids such as oleic acid find use in the preparation of injectables.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

The renin-inhibiting compounds of this invention may be administered in combination with other agents used in antihypertensive therapy such as diuretics, α and/or β-adrenergic blocking agents, CNS-acting agents, adrenergic neuron blocking agents, vasodilators, angiotensin I converting enzyme inhibitors, and the like as described for example in published European patent application 156,318.

The present invention is also directed to combinations of the novel renin-inhibitory peptides of Formula I with one or more antihypertensive agents selected from the group consisting of diuretics, α and/or β-adrenergic blocking agents, CNS-acting agents, adrenergic neuron blocking agents, vasodilators, angiotensin I converting enzyme inhibitors, and other antihypertensive agents.

For example, the compounds of this invention can be given in combination with such compounds or salt or other derivative forms thereof as:
Diuretics: acetazolamide; amiloride; bendroflumethiazide; benzthia zide; bumetanide; chlorothiazide. chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynaten; trimaterene; trichlormethiazide;
α-Adrenergic Blocking Agents: dibenamine; phentolamine; phenoxybenzamine; prazosin; tolazoline;
β-Adrenergic Blocking Agents: atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl)(befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl)(bevantolol);
(((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]-propoxy)-indole);
(carbazolyl-4-oxy-5,2-(2-methoxyphenoxy)-ethylamino-2-propanol);
(1-((1,1-dimethylethyl)amino)-3-((2-methyl 'H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)-amino]propoxy]benzonitrile HCl) (bucindolol);
(α-[(tert.butylamino)methyl]-7-ethyl-2-benzofuranmethanol) (bufuralol);
(3-[3-acetyl-4-[3-(tert.butylamino)-2-hydroxypropyl]-phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)-acetanilide HCl) (diacetolol);
(methyl-4-[2-hydroxy-3-[(1-methylethyl)aminopropoxyl]]-benzenepropanoate HCl) (esmolol);
(erythro-DL-1-(7-methylindan-4-yloxy)-3-isopropylaminobutan-2-ol);
(1-(tert.butylamino)-3-[0-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)amino]ethyl]benzamide;
(4-hydroxy-9-[2-hydroxy-3-(isopropylamino)-propoxy]-7-methyl-5H-furo[3,2-g][1]-benzopyran-5-one)(iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropylaminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-(2-hydroxy-3-[(1-methylethyl)amino]propoxyphenyl]butanamide) (acebutolol);
((±)-4′-[3-(tert-butylamino)-2-hydroxypropoxy]spiro[cyclohexane-1,2′-indan]-1′-one) (spirendolol);
(7-[3-[[2-hydroxy-3-[(2-methylindol-4-yl)oxylpropyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.-butylamino)-2-hydroxy-propoxy)benzonitrile HCl) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbotyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol) (nadolol);
((S)-1-(2-cyclopentylphenoxy)-3-[(1,1-dimethylethyl)amino]-2-propanol sulfate (2:1)) (penbutolol);
(4′-[1-hydroxy-2-(amino)ethyl]methanesulfonanilide) (sotalol);
(2-methyl-3-[4-(2-hydroxy-3-tert.butylaminopropoxy)phenyl]-7-methoxyisoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) ( indopanolol);
((±)-6-[[2-[[3-(p-butoxyphenoxy)-2-hydroxypropyl]amino]ethyl]amino]-1,3-dimethyluracil)(pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);
Angiotensin I Converting Enzyme Inhibitors:
1-(3-mercapto-2-methyl-1-oxopropyl)-L-proline (captopril);
(1-(4-ethoxycarbonyl-2,4(R,R)-dimethylbutanoyl)indoline-2(S)-car boxylic acid);
(2-[2-[(1-(ethoxycarbonyl)-3-phenyl-propyl]amino]-1-oxopropyl]-1,2,3,4-tetrahydro-3-isoquinoline carboxylic acid);
((S)-1-[2-[(1-ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)-cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);
(N(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
N²-[1-(S)-carboxy-3-phenylpropyl]-L-lysyl-L-proline (lysinopril);
Other Antihypertensive Agents: aminophylline; cryptenamine acetates and tannates; deserpidine; meremethoxylline procaine; pargyline; trimethaphan camsylate; and the like, as well as admixtures and combinations thereof.

Typically, the individual daily dosages for these combinations can range from about one-fifth of the minimally recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

The novel peptides of the present invention possess an excellent degree of activity in treating renin-associated hypertension and hyperaldosteronism.

Renin inhibitors have also been disclosed to control the rise in intraocular pressure associated with the use of steroidal anti-inflammatory drugs as described in International Application PCT/US86/02291 (International Publication Number WO 87/02581 dated 7 May 1987).

The compounds of the present invention are prepared as depicted in the charts and as described more fully in the Preparations and Examples.

The process of the present invention is more completely under stood by reference to the charts below. In these charts, the variables are as defined above.

### CHART A

Chart A describes the representative preparation of the renin inhibitory peptides of the present invention. In Chart A, X is H or H-Pro; Y is a direct bond or Pro; and Z is Ile-Amp or Mba. LVA is Leuψ[CH0HCH₂]Val; Amp is 2-aminomethylpyridine; Mba is 2S-methylbutylamine.

The compound of formula A-1 is suleptanic acid triethylamine salt, which is readily available, and the compounds of formula A-2, (wherein X is H, Y is a direct bond and Z is Ile-Amp; wherein X is H-Pro, Y is Pro and Z is Ile-Amp; and wherein X is H, Y is a direct bond and Z is Mba,) are precursors or parts of known renin inhibitors.

Direct conjugation of the compounds of formula A-2 with suleptanic acid of formula A-1 using a dehydrating agent, such as diethylphosphoryl cyanide, affords material that can be chromatographed directly on silica gel. partition of the material between aqueous sodium sulfate and n-butanol gives the corresponding sodium salts of formula A-3, which are highly water soluble and can be lyophilized to white powders.

### CHART B

Chart B describes the preparation of intermediates used to make the following renin inhibitory peptides: N,N′-N-Methyltaurine, L-0-methyltyrosyl-L-histidyl-2S-amino-1-cyclohexyl-3R,4S-dihydroxy-6-methylheptane, octanoic acid diamide, sodium salt; N,N′-N-Methyltaurine, L-phenylalanyl-N^{α}-methyl-L-histidyl-2S-amino-1-cyclohexyl-3R,4S-dihydroxy-6-methylheptane, octanoic acid diamide, sodium salt; N,N′-N-Methyltaurine, L-prolyl-L-0-methyltyrosyl-L-histidyl-2S-amino-1-cyclohexyl-3R,4S-dihydroxy-6-methylheptane, octanoic acid diamide, sodium salt; N,N′-N-Methyltaurine, L-prolyl-L-phenylalanyl-N^{α}-methyl-L-histidyl-2S-amino-1-cyclohexyl-3R,4S-dihydroxy-6-methylheptane, octanoic acid diamide, sodium salt. Published European patent application 0189203 (Abbott) discloses the hydrochloride salt of the intermediate compound of formula B-5.

The Grignard reagent, generated from 1-bromo-2-methylpropene and magnesium turnings, is added to the aldehyde of formula B-1, PCT patent application, Serial No. 00307, filed 23 November 1987, to give two epimers of formula B-2 and B-3 which are separated by column chromatography. The unsaturated alcohol of formula B-3 is then hydrogenated over platinum on charcoal to give the compound of formula B-4. The protecting groups on the compound of formula B-4 are removed by treatment with hydrogen chloride in methanol, and after neutralization with sodium carbonate, the free amine of formula B-5 is isolated.

### CHART C

Chart C describes the preparation of intermediates used to make the renin inhibitory peptide N,N′-N-Methyltaurine, L-phenylalanyl-N^{α}-methyl-L-histidyl-2S-amino-1-cyclohexyl-3R,4S-dihydroxy-6-methylheptane, octanoic acid diamide, sodium salt.

The compound of formula B-5 from Chart B is used as the C-1 starting material. The amine of formula C-1 is coupled to N-tert-butyloxycarbonyl-N-methyl-N-tosyl-L-histidine to give the compound of formula C-2. The tert-butyloxycarbonyl protecting group is removed with trifluoroacetic acid and the resulting amine is then coupled to N-tert-butyloxycarbonyl-L-phenylalanine to give the compound of formula C-3. The tosyl protecting group is removed with 1-hydroxybenzotriazole to give the compound of formula C-4.

The tert-butyloxycarbonyl protecting group is removed with trifluoroacetic acid and the resulting amine is then coupled to suleptanic acid to give the desired renin inhibitory peptide.

### CHART D

Chart D describes the preparation of intermediates used to make the renin inhibitory peptide N,N′-N-Methyltaurine, L-prolyl-L-phenylalanyl-N^{α}-methyl-L-histidyl-2S-amino-1-cyclohexyl-3R,4S-dihydroxy-6-methylheptane, octanoic acid diamide, sodium salt.

The compound of formula C-3 from Chart C is used as the D-1 starting material. The tert-butyloxycarbonyl protecting group is removed from the compound of formula D-1 with trifluoroacetic acid and the resulting amine is then coupled to N-tert-butyloxycarbonyl-L-proline to give the compound of formula D-2. The tosyl protecting group is removed with 1-hydroxybenzotriazole to give the compound of formula D-3.

The tert-butyloxycarbonyl protecting group is removed with trifluoroacetic acid and the resulting amine is then coupled to suleptanic acid to give the desired renin inhibitory peptide.

### CHART E

Chart E describes the preparation of intermediates used to make the renin inhibitory peptide N,N′-N-Methyltaurine, L-0-methyltyrosyl-L-histidyl-2S-amino-1-cyclohexyl-3R,4S-dihydroxy-6-methylheptane, octanoic acid diamide, sodium salt.

The compound of formula B-3 from Chart B is used as the E-1 starting material. The amine of formula E-1 is coupled to N-tert-butyloxycarbonyl-N-tosyl-L-histidine to give the compound of formula E-2. The tert-butyloxycarbonyl protecting group is removed with trifluoroacetic acid and the resulting amine is then coupled to N-tert-butyloxycarbonyl-0-methyl-L-tyrosine to give the compound of formula E-3. The tosyl protecting group is removed with 1-hydroxybenzotriazole to give the compound of formula E-4.

The tert-butyloxycarbonyl protecting group is removed with trifluoroacetic acid and the resulting amine is then coupled to suleptanic acid to give the desired renin inhibitory peptide.

### CHART F

Chart F describes the preparation of intermediates used to make the renin inhibitory peptide N,N′-N-Methyltaurine, L-prolyl-L-0-methyltyrosyl-L-histidyl-2S-amino-1-cyclohexyl-3R,4S-dihydroxy-6-methylheptane, octanoic acid diamide, sodium salt.

The compound of formula E-3 from Chart E is used as the F-1 starting material. The tert-butyloxycarbonyl protecting group is removed from the compound of formula F-1 with trifluoroacetic acid and the resulting amine is then coupled to N-tert-butyloxycarbonyl-L-proline to give the compound of formula F-2. The tosyl protecting group is removed with 1-hydroxybenzotriazole to give the compound of formula F-3.

The tert-butyloxycarbonyl protecting group is removed with trifluoroacetic acid and the resulting amine is then coupled to suleptanic acid to give the desired renin inhibitory peptide.

Generally, the renin inhibiting polypeptides may be prepared by solution phase peptide synthetic procedures analogous to those described hereinafter or to those methods known in the art. Appropriate protecting groups, reagents, and solvents for the solution phase method can be found in "The Peptides: Analysis, Synthesis, and Biology," Vols. 1-5, eds. E. Gross and T. Meienhofer, Academic Press, NY, 1979-1983; "The Practice of Peptide Synthesis", M. Bodansky and A. Bodansky, Springer-Verlag, New York, 1984; "The Principles of Peptide Synthesis", M. Bodansky, Springer-Verlag, New York, 1984. Thus, for example, the carboxylic moiety of N^{α}-t-butyloxycarbonyl (Boc)-substituted amino acid derivatives having suitable side chain protecting groups, if necessary, may be condensed with the amino functionality of a suitably protected amino acid or peptide using a conventional coupling protocol such as dicyclohexylcarbodiimide (DCC) and 1-hydroxybenzotriazole (HOBT) or diethylphosphoryl cyanide (DEPC) and triethylamine (Et₃N) in methylene chloride or dimethylformamide.

Following coupling reaction completion, the N^{α}-Boc moiety may be selectively removed with 50% trifluoroacetic acid with or without 2% anisole (v/v) in methylene chloride. Neutralization of the resultant trifluoroacetate salt may be accomplished with 10% diisopropylethylamine or sodium bicarbonate in methylene chloride.

Variations in the above description for starting materials, reactants, reaction conditions and required protecting groups to obtain other such N-alkylated compounds are known to an ordinarily skilled chemist or are readily available in the literature.

The compounds of the present invention may be in either free form or in protected form at one or more of the remaining (not previously protected) peptide, carboxyl, amino, hydroxy, or other reactive groups. The protecting groups may be any of those known in the polypeptide art. Examples of nitrogen and oxygen protection groups are set forth in T.W. Greene, Protecting Groups in Organic Synthesis, Wiley, New York, (1981); J.F.W. McOmie, ed. Protective Groups in Organic Chemistry, Plenum Press (1973); and J. Fuhrhop and G. Benzlin, Organic Synthesis, Verlag Chemie (1983). Included among the nitrogen protective groups are t-butoxycarbonyl (Boc), benzyloxycarbonyl, acetyl, allyl, phthalyl, benzyl, benzoyl, trityl and the like.

The following compounds of the present invention are preferred:
N,N′-N-Methyltaurine, L-phenylalanyl-N^{α}-methyl-L-histidyl-5S-amino-4S-hydroxy-2S-isopropyl-7-methyloctanoyl-L-isoleucyl-2-pyridyl-methylamine, octanoic acid diamide, sodium salt;
N,N′-N-Methyltaurine, L-phenylalanyl-N^{α}-methyl-L-histidyl-5S-amino-4S-hydroxy-2S-isopropyl-7-methyloctanoyl-2S-methylbutylamine, octanoic acid diamide, sodium salt;
N,N′-N-Methyltaurine-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, octanoic acid diamide, sodium salt;
N,N′-N-Methyltaurine, L-0-methyltyrosyl-L-histidyl-2S-amino-1-cyclohexyl-3R,4S-dihydroxy-6-methylheptane, octanoic acid diamide, sodium salt;
N,N′-N-Methyltaurine, L-phenylalanyl-N^{α}-methyl-L-histidyl-2S-amino-1-cyclohexyl-3R,4S-dihydroxy-6-methylheptane, octanoic acid diamide, sodium salt;
N,N′-N-Methyltaurine, L-prolyl-L-phenylalanyl-N^{α}-methyl-L-histidyl-5S-amino-4S-hydroxy-2S-isopropyl-7-methyloctanoyl-L-isoleucyl-2-pyridylmethylamine, octanoic acid diamide, sodium salt;
N,N′-N-Methyltaurine, L-prolyl-L-phenylalanyl-N^{α}-methyl-L-histidyl-2S-amino-1-cyclohexyl-3R,4S-dihydroxy-6-methylheptane, octanoic acid diamide, sodium salt;
N,N′-N-Methyltaurine, L-prolyl-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, octanoic acid diamide, sodium salt;
N,N′-N-Methyltaurine, L-prolyl-L-0-methyltyrosyl-L-histidyl-2S-amino-1-cyclohexyl-3R,4S-dihydroxy-6-methylheptane, octanoic acid diamide, sodium salt.

The most preferred compounds are:
N,N′-N-Methyltaurine, L-prolyl-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, octanoic acid diamide, sodium salt;
N,N′-N-Methyltaurine, L-prolyl-L-0-methyltyrosyl-L-histidyl-2S-amino-1-cyclohexyl-3R,4S-dihydroxy-6-methylheptane, octanoic acid diamide, sodium salt;
N,N′-N-Methyltaurine, L-prolyl-L-phenylalanyl-N^{α}-methyl-L- histidyl-2S-amino-1-cyclohexyl-3R,4S-dihydroxy-6-methylheptane, octanoic acid diamide, sodium salt;
N,N′-N-Methyltaurine, L-prolyl-L-phenylalanyl-N^{α}-methyl-L-histidyl-5S-amino-4S-hydroxy-2S-isopropyl-7-methyloctanoyl-L-isoleucyl-2-pyridylmethylamine, octanoic acid diamide, sodium salt;
N,N′-N-Methyltaurine, L-phenylalanyl-N^{α}-methyl-L-histidyl-5S-amino-4S-hydroxy-2S-isopropyl-7-methyloctanoyl-2S-methylbutylamine, octanoic acid diamide, sodium salt.

### DESCRIPTION OF THE PREFERRED EMBODIMENTS

The following Preparations and Examples illustrate the present invention.

In the Preparations and Examples below and throughout this document:
¹H-NMR is nuclear magnetic resonance
Amp is 2-(aminomethyl)pyridinyl
Bn is benzylester
BOC is t-butoxycarbonyl
Bz is benzyl
C is centigrade
Cbz is benzyloxycarbonyl
CDCl₃ is deuteriochloroform
Celite is a filter aid.
DCC is dicyclohexylcarbodiimide
DEPC is diethylphosphoryl cyanide
ET0Ac is ethyl acetate
FTrp is Nⁱⁿ⁻formyl-Trp
g is grams
His is histidine
HOBT is 1-hydroxybenzotriazole
HPLC is high performance liquid chromatography
Iba is isobutylamine
Ile is isoleucine
IR is infrared spectra
LVA is Leuψ(CH(OH)CH₂)Val with the S configuration at C4 (the hydroxyl-bearing carbon atom).
M or mol is mole
Me is methyl
min is minute
ml is milliliter
MPLC is medium pressure liquid chromatography
MS is mass spectroscopy
Ph is phenyl
Phe is phenylalanine
RIP means a compound having the formula H-Pro-His-Phe-His-Phe-Phe-Val-Tyr-Lys-0H.2(CH₃C(0)0H).XH₂0 which is a known renin-inhibiting peptide.
Sta is statine
TBS is tert-butyldimethylsilyl
TEA is triethylamine
TFA is trifluoroacetic acid
THF is tetrahydrofuran
TLC is thin layer chromatography
Tos is p-toluenesulfonyl
Ts0H is p-toluenesulfonic acid.

The wedge-shape line indicates a bnd which extends above the plane of the paper relative to the plane of the compound thereon.

The dotted line indicates a bond which extends below the plane of the paper relative to the plane of the compound thereon.

In the examples below, HPLC is high pressure liquid chromatography and k′ is the partition ratio obtained. The solvent system used is indicated in parentheses after the partition ratio: A is 50% methanol, 50% aqueous phosphate pH 3 buffer; B is 55% methanol, 45% aqueous phosphate pH 3 buffer; C is 60% methanol, 40% aqueous phosphate pH 3 buffer; and D is 65% methanol, 35% aqueous phosphate pH 3 buffer. The flow rates were at 1.5 ml/min. The detector was set at 225 or 254 nm.

In the examples below, the in vitro IC₅₀ is measured in nano-molars. The in vitro test is performed as described in U.S. patent application, Serial No. 147,073, filed 20 January 1988, and in published European patent application 0173481, which are hereby incorporated by reference. Compounds of the present invention have also exhibited renin-inhibitory activity during in vivo testing.

### Example 1 N-Methyltaurine, L-phenylalanyl-N^{α}-methyl-L-histidyl-5S-amino-4S-hydroxy-2S-isopropyl-7-methyloctanoyl-L-isoleucyl-2-pyridylmethylamine, octanoic acid diamide, sodium salt

To a stirred solution of 1.6 g of L-phenylalanyl-N^{α}-methyl-L-histidyl-5S-amino-4S-hydroxy-2S-isopropyl-7-methyloctanoyl-L-isoleucyl-2-pyridylmethylamine disclosed in U.S. Patent Application, Serial No. 147,073, filed 20 January 1988, in 7 ml of dichloromethane is added 3.6 ml of a 0.652M solution of suleptanic acid, triethylammonium salt, in acetonitrile, followed by 0.84 ml of diisopropylethylamine and 0.37 ml of diethylphosphoryl cyanide. After stirring overnight, the concentrated mixture is chromatographed on silica gel with 10%-30% methanol (saturated with ammonia) in dichloromethane. The residue is dissolved in 20 ml of water and 2 g of sodium sulfate is added. The resulting aqueous phase is extracted with two 30 ml portions of n-butanol. The combined organic phase is concentrated and the residue dissolved in 30 ml of water and then filtered. The filtrate is lyophilized to give 1.52 g of the title product.

Physical characteristics are as follows:
MS:[M+H]⁺ at m/z = 1010.583.
HPLC:k′ = 9.02 (D).
In vitro IC₅₀ = 6.6 x 10⁻¹⁰.

### Example 2 N-Methyltaurine, L-prolyl-L-phenylalanyl-N^{α}-methyl-L-histidyl-5S-amino-4S-hydroxy-2S-isopropyl-7-methyloctanoyl-L-isoleucyl-2-pyridylmethylamine, octanoic acid diamide, sodium salt

Following the procedures described in Example 1, but using L-prolyl-L-phenylalanyl-N^{α}-methyl-L-histidyl-5S-amino-4S-hydroxy-2S-isopropyl-7-methyloctanoyl-L-isoleucyl-2-pyridylmethylamine, disclosed in U.S. Patent Application, Serial No. 147,073, filed 20 January 1988, in place of the peptide used in Example 1, the title product is obtained.

Physical characteristics are as follows:
MS:[M+H]⁺ at m/z = 1129.614.
HPLC:k′ = 11.80 (D).
In vitro IC₅₀ = 1.8 x 10⁻⁹.

### Example 3 N-Methyltaurine, L-phenyl-alanyl-N^{α}-methyl-L-histidyl-5S-amino-4S-hydroxy-2S-isopropyl-7-methyloctanoyl-2S-methylbutylamine, octanoic acid diamide, sodium salt

Following the procedures described in Example 1, but using L-phenyl-alanyl-N^{α}-methyl-L-histidyl-5S-amino-4S-hydroxy-2S-isopropyl-7-methyloctanoyl-2S-methylbutylamine, disclosed in U.S. Patent Application, Serial No.147,073, filed 20 January 1988, in place of the peptide used in Example 1, the title product is obtained.

Physical characteristics are as follows:
MS[M+H]⁺ at m/z = 876.5290.
HPLC:k′ = 8.93 (D).
In vitro IC₅₀ = 2.1 x 10⁻⁹.

### Example 4 N,N′-N-Methyltaurine, L-phenylalanyl-β-L-aspartyl-5S-amino-4S-hydroxy-2S-isopropyl-7-methyloctanoyl-2S-methylbutylamine, octanoic acid diamide, disodium salt

Following the procedures described in Example 1, but using L-phenylalanyl-β-L-aspartyl-5S-amino-4S-hydroxy-2S-isopropyl-7-methyloctanoyl-2S-methylbutylamine, disclosed in U.S. Patent Application, Serial No. 121,270, filed 16 November 1987, in place of the peptide used in Example 1, the title product is obtained.

Physical characteristics are as follows:
HPLC : k′ = 1.98 (C).
FAB-MS Found = 884.4462.
In vitro IC₅₀ = 5.4 x 10⁻⁸.

### Example 5 N,N′-N-Methyltaurine-L-phenylalanyl-β-L-aspartyl-5S-amino-4S-hydroxy-2S-isopropyl-7-methyloctanoyl-L-isoleucyl-2-pyridylmethylamine, octanoic acid diamide, sodium salt

Following the procedures described in Example 1, but using L-phenylalanyl-β-L-aspartyl-5S-amino-4S-hydroxy-2S-isopropyl-7-methyloctanoyl-L-isoleucyl-2-pyridylmethylamine, disclosed in U.S. Patent Application, Serial No. 121,270, filed 16 November 1987, in place of the peptide used in Example 1, the title product is obtained.

Physical characteristics are as follows:
HPLC : k′ = 1.66 (C).
FAB-MS Found = 1012.484.
In vitro IC₅₀ = 5.4 x 10⁻¹⁰.

### Example 6 N,N′-N-Methyltaurine-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, octanoic acid diamide, sodium salt

Following the procedures described in Example 1, but using L-phenylalanyl-N^{α}-methyl-L-histidyl-5S-amino-4S-hydroxy-2S-isopropyl-7-methyloctanoyl-L-isoleucyl-1-oxa-2-pyridylmethylamine, disclosed in U.S. Patent Application, Serial No. 151,129, filed 1 February 1988, in place of the peptide used in Example 1, the title product is obtained.

Physical characteristics are as follows:
HPLC : k′ = 15.61 (B).
FAB-MS Found = 1048.554.
In vitro IC₅₀ = 1.2 x 10⁻⁹.

### Example 7 N,N′-N-methyltaurine, L-0-methyltyrosyl-L-histidyl-2S-amino-1-cyclohexyl-3R,4S-dihydroxy-6-methylheptane, octanoic acid diamide, sodium salt

Following the procedures described in Example 1, but using L-0-methyltyrosyl-L-histidyl-2S-amino-1-cyclohexyl-3R,4S-dihydroxy-6-methylheptane, disclosed in published European patent application 0189203 (Abbott) in place of the peptide used in Example 1, the title product is obtained.

Physical characteristics are as follows:
HPLC : k′ = 8.59 (D).
FAB-MS Found = 857.4490.
In vitro IC₅₀ = 1.6 x 10⁻⁹.

### Example 8 N,N′-N-Methyltaurine, L-phenylalanyl-N^{α}-methyl-L-histidyl-2S-amino-1-cyclohexyl-3R,4S-dihydroxy-6-methylheptane, octanoic acid diamide, sodium salt

Following the procedures described in Example 1, but using L-phenylalanyl-N^{α}-methyl-L-histidyl-2S-amino-1-cyclohexyl-3R,4S-dihydroxy-6-methylheptane, disclosed in published European patent application 0189203 (Abbott) in place of the peptide used in Example 1, the title product is obtained.

Physical characteristics are as follows:
HPLC : k′ = 8.72 (D).
FAB-MS Found = 841.4528.
In vitro IC₅₀ = 1.3 x 10⁻⁹.

### Example 9 N,N′-N-Methyltaurine, L-phenylalanyl-β-L-aspartyl-5S-amino-4S-hydroxy-2S-isopropyl-7-methyloctanoyl-L-isoleucyl-1-oxa-2-pyridylmethylamine, octanoic acid diamide, sodium salt

Following the procedures described in Example 1, but using L-phenylalanyl-β-L-aspartyl-5S-amino-4S-hydroxy-2S-isopropyl-7-methyloctanoyl-L-isoleucyl-1-oxa-2-pyridylmethylamine, disclosed in U.S. Patent Publication, Serial No. 121,270, filed 16 November 1987, in place of the peptide used in Example 1, the title product is obtained.

Physical characteristics are as follows:
HPLC : k′ = 2.89 (A).
FAB-MS Found = 1012 (No high resolution MS was obtained.)
In vitro IC₅₀ = 2.0 x 10⁻⁸.

### Example 10 N,N′-N-Methyltaurine, L-phenylalanyl-L-histidyl-4S-amino-5-cyclohexyl-2,2-difluoro-3R-hydroxypentanoyl-L-isoleucyl-2-pyridylmethylamine, octanoic acid diamide, sodium salt

Following the procedures described in Example 1, but using L-phenylalanyl-L-histidyl-4S-amino-5-cyclohexyl-2,2-difluoro-3R-hydroxy-pentanoyl-L-isoleucyl-2-pyridylmethylamine, disclosed in U.S. Patent Application, Serial No. 934,540, filed 28 November 1986, in place of the peptide used in Example 1, the title product is obtained.

Physical characteristics are as follows:
HPLC : k′ = 9.98 (D).
FAB-MS Found = 1016.510.
In vitro IC₅₀ = 5.1 x 10⁻⁹.

### Example 11 N,N′-N-Methyltaurine, L-prolyl-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, octanoic acid diamide, sodium salt

Following the procedures described in Example 1, but using L-prolyl-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, disclosed in U.S. Patent Application, Serial No. 151,129, filed 1 February 1988, in place of the peptide used in Example 1, the title product is obtained.

Physical characteristics are as follows:
HPLC : k′ = 21.45 (B).
FAB-MS Found = 1145.603.
In vitro IC₅₀ = 3.1 x 10⁻¹⁰.

### Example 12 N,N′-N-Methyltaurine, L-prolyl-L-0-methyltyrosyl-L-histidyl-2S-amino-1-cyclohexyl-3R,4S-dihydroxy- 6-methylheptane, octanoic acid diamide, sodium salt

Following the procedures described in Example 1, but using L-prolyl-L-0-methyltyrosyl-L-histidyl-2S-amino-1-cyclohexyl-3R,4S-dihydroxy-6-methylheptane, disclosed in published European patent application 0189203 (Abbott) in place of the peptide used in Example 1, the title product is obtained.

Physical characteristics are as follows:
HPLC : k′ = 11.03 (D).
FAB-MS Found = 954.4984.
In vitro IC₅₀ = 8.6 x 10⁻¹¹.

### Example 13 N,N′-N-Methyltaurine, L-prolyl-L-phenylalanyl-N^{α}-methyl-L-histidyl-2S-amino-1-cyclohexyl-3R,4S-dihydroxy-6-methylheptane, octanoic acid diamide, sodium salt

Following the procedures described in Example 1, but using L-prolyl-L-phenylalanyl-N^{α}-methyl-L-histidyl-2S-amino-1-cyclohexyl-3R-4S-dihydroxy-6-methylheptane, disclosed in published European patent application 0189203 (Abbott) in place of the peptide used in Example 1, the title product is obtained.

Physical characteristics are as follows:
HPLC : k′ = 9.77 (D).
FAB-MS Found = 916.5237.
In vitro IC₅₀ = 2.0 x 10⁻¹⁰.

### Example 14 N,N′-N-Methyltaurine, L-prolyl-L-phenylalanyl-β-L-aspartyl-5S-amino-4S-hydroxy-2S-isopropyl-7-methyloctanoyl-L-isoleucyl-2-pyridylmethylamine, octanoic acid diamide, sodium salt

Following the procedures described in Example 1, but using L-prolyl-L-phenylalanyl-β-L-aspartyl-5S-amino-4S-hydroxy-2S-isopropyl-7-methyloctanoyl-L-isoleucyl-2-pyridylmethylamine, disclosed in U.S. Patent Application, Serial No. 121,270, filed 16 November 1987, in place of the peptide used in Example 1, the title product is obtained.

Physical characteristics are as follows:
HPLC : k′ = 5.67 (A).
FAB-MS Found = 1109.532.
In vitro IC₅₀ = 2.0 x 10⁻⁹.

### Example 15 N,N′-N-Methyltaurine, L-prolyl-L-phenylalanyl-β-L-aspartyl-5S-amino-4S-hydroxy-2S-isopropyl-7-methyloctanoyl-L-isoleucyl-1-oxa-2-pyridylmethylamine, octanoic acid diamide, sodium salt

Following the procedures described in Example 1, but using L-prolyl-L-phenylalanyl-β-L-aspartyl-5S-amino-4S-hydroxy-2S-isopropyl-7-methyloctanoyl-L-isoleucyl-1-oxa-2-pyridylmethylamine, disclosed in U.S. Patent Application, Serial No. 121,270, filed 16 November 1987, in place of the peptide used in Example 1, the title product is obtained.

Physical characteristics are as follows:
HPLC : k′ = 5.01 (A).
FAB-MS Found = 1109.554.
In vitro IC₅₀ = 5.1 x 10⁻⁹.

### Example 16 N,N′-N-Methyltaurine, L-phenylalanyl-L-histidyl-4S-amino-5-cyclohexyl-2,2-difluoro-3-oxo-pentanoyl-L-isoleucyl-2-pyridylmethylamine, octanoic acid diamide, sodium salt.

Following the procedures described in Example 1, but using L-phenylalanyl-L-histidyl-4S-amino-5-cyclohexyl-2,2-difluoro-3-oxopentanoyl-L-isoleucyl-2-pyridylmethylamine, disclosed in U.S. Patent Application, Serial No. 934,540, filed 28 November 1986, in place of the peptide used in Example 1, the title product is obtained.

### Preparation 1 1R and 1S-[3-tert-Butyloxycarbonyl-4S-cyclohexylmethyl-2,2-dimethyl-5R-oxazolidinyl]-3-methyl-2-butene-1-ol (Formula B-2 and B-3) Refer to Chart B.

To a stirred mixture of 0.54 g of magnesium turnings and a crystal of iodine in 10 ml of dry tetrahydrofuran under argon in an oil bath at 80°C is slowly added a solution of 2.84 g of 1-bromo-2-methylpropene in 5 ml of tetrahydrofuran. After 3 h, the reaction mixture is allowed to cool and then is stirred in an ice bath. A solution of 4.6 g of 3-tert-butyloxycarbonyl-4S-cyclohexylmethyl-2,2′-dimethyl-5R-oxazolidinyl carboxaldehyde in 10 ml of tetrahydrofuran is slowly added. After 1 h, the reaction mixture is allowed to warm to room temperature and stirred overnight. The mixture is treated with saturated aqueous ammonium chloride and then extracted with dichloromethane. The organic phase is dried (magnesium sulfate) and then concentrated. The residue is flashed chromatographed on silica gel with 10%-15% ethyl acetate in hexane to give 1.34 g of the title product B-2 and 2.88 g of the title product B-3.

Physical characteristics of B-2 are as follows:
¹H-NMR: 1.48, 1.57, 1.62, 1.73, 1.78, 5.27.
Physical characteristics of B-3 are as follows:
¹H-NMR: 1.45, 1.47, 1.50, 1.73, 1.77, 5.12.

### Preparation 2 1S-[3-tert-Butyloxycarbonyl-4S-cyclohexylmethyl-2,2-dimethyl-5R-oxazolidinyl]-3-methyl-1-butanol (Formula B-4) Refer to Chart B.

To a solution of 2.88 g of the title product B-3 of Preparation 1 in 20 ml of ethyl acetate is added 0.15 g of 5% platinum on charcoal. This mixture is shaken on a Parr shaker under 50 psi of hydrogen for 5 h. The resulting mixture is filtered through Celite with ethyl acetate washings and the filtrate is concentrated to give 2.5 g of the title product.

Physical characteristics are as follows:
¹H-NMR: 0.92, 0.97, 1.48, 1.52, 1.55.

### Preparation 3 2S-Amino-1-cyclohexyl-3R,4S-dihydroxy-6-methylheptane (Formula B-5) Refer to Chart B.

A solution of 2.5 g of the title product of Preparation 2 in 15 ml of 1M hydrochloric acid in methanol is allowed to stir at room temperature for 3 h. Small portions of 2.5 g of solid sodium bicarbonate is slowly added. After stirring for 15 min, the mixture is diluted with dichloromethane and then filtered through Celite with dichloromethane washings. The filtrate is concentrated and the resulting residue is flash chromatographed on silica gel with 5%-10% methanol (saturated with gaseous ammonia) in dichloromethane to give 1.3 g of the title product.

Physical characteristics are as follows:
¹H-NMR: 0.92, 0.97, 3.05, 3.25, 3.8.

### Preparation 4 N-tert-Butyloxycarbonyl-N^{α}-methyl-N^{im}-tosyl-L-histidyl-2S-amino-1-cyclohexyl-3R,4S-dihydroxy-6-methylheptane (Formula C-2) Refer to Chart C.

To a stirred solution of 0.87 g of N-tert-butyloxycarbonyl-N^{α}-methyl-N^{im}-tosyl-L-histidine and 0.384 g of the title product of Preparation 3 in 8 ml of dichloromethane is added 0.75 ml of diisopropylethylamine, followed by 0.33 ml of diethylphosphoryl cyanide. After 3 h, the concentrated reaction mixture is flash chromatographed on silica gel with 30%-40% ethyl acetate in dichloromethane to give 0.605 g of the title product.

Physical characteristics are as follows:
¹H-NMR: 0.83, 0.92, 1.43, 2.44, 2.76, 7.07, 7.36, 7.79, 7.90.

### Preparation 5 N-tert-Butyloxycarbonyl-L-phenylalanyl-N^{α}-methyl-N^{im}-tosyl-L-histidyl-2S-amino-1-cyclohexyl-3R,4S-dihydroxy-6-methylheptane (Formula C-3) Refer to Chart C.

A solution of 0.605 g of the title product of Preparation 4 in 4 ml of 1:1 = dichloromethane:trifluoroacetic acid is allowed to stir for 45 min. The mixture is then slowly added to 3 g of sodium bicarbonate in 40 ml of water. The resulting mixture is extracted with dichloromethane. The organic phase is dried (magnesium sulfate) and then concentrated to give 0.52 g of the corresponding amine.

To a stirred solution of this material and 0.38 g of N-tert-butyloxycarbonyl-L-phenylalanine in 4 ml of dichloromethane is added 0.32 ml of diisopropylethylamine, followed by 0.22 ml of diethylphosphoryl cyanide. After 15 h, the concentrated reaction mixture is flash chromatographed on silica gel with 30%-50% ethyl acetate in dichloromethane to give 0.44 g of the title product.

Physical characteristics are as follows:
¹H-NMR: 0.81, 0.90, 1.37, 2.42, 2.80.

### Preparation 6 N-tert-Butyloxycarbonyl-L-phenylalanyl-N^{α}-methyl-L-histidyl-2S-amino-1-cyclohexyl-3R,4S-dihydroxy-6-methylheptane (Formula C-4) Refer to Chart C.

A solution of 63 mg of the title product of Preparation 5 and 43 mg of 1-hydroxybenzotriazole in 0.5 ml of methanol is allowed to stir overnight. The concentrated mixture is chromatographed on silica gel with 5% methanol (saturated with gaseous ammonia) in dichloromethane to give 40 ml of the title product.

Physical characteristics are as follows:
FAB-MS: 642.4237 (Found).
HPLC: k′=10.22 (90:10 = methanol:phosphate pH 3 buffer).

### Preparation 7 N-tert-Butyloxycarbonyl-L-prolyl-L-phenylalanyl-N^{α}-methyl-N^{im}-tosyl-L-histidyl-2S-amino-1-cyclohexyl-3R,4S-dihydroxy-6-methylheptane (Formula D-2) Refer to Chart D.

By the same procedure as in the preparation of the title product of Preparation 5, 0.37 g of the title product of Preparation 5 is deprotected with 1:1 = dichloromethane:trifluoroacetic acid and then coupled to N-tert-butyloxycarbonyl-L-proline to give 0.345 g of the title product.

Physical characteristics are as follows:
¹H-NMR: 0.80, 0.91, 1.40, 2.42, 2.80.

### Preparation 8 N-tert-Butyloxycarbonyl-L-prolyl-L-phenylalanyl-N^{α}-methyl-L-histidyl-2S-amino-1-cyclohexyl-3R,4S-dihydroxy-6-methylheptane (Formula D-3) Refer to Chart D.

By the same procedure as in the preparation of the title product of Preparation 6, 0.74 mg of the title product of Preparation 7 and 57 mg of 1-hydroxybenzotriazole gives 57 mg of the title product.

Physical characteristics are as follows:
FAB-MS: 739.4728 (Found).
HPLC: k′=11.93 (90:10 = methanol:phosphate pH 3 buffer).

The following is a possible concordance of US Patent Application Serial Numbers given above, and publications/applications:
USSN 147,073 = EP-A-0173481
USSN 121,270 = WO-A-8904833
USSN 151,129 = PCT/US89/00247
USSN 934,540 = WO-A-8606379

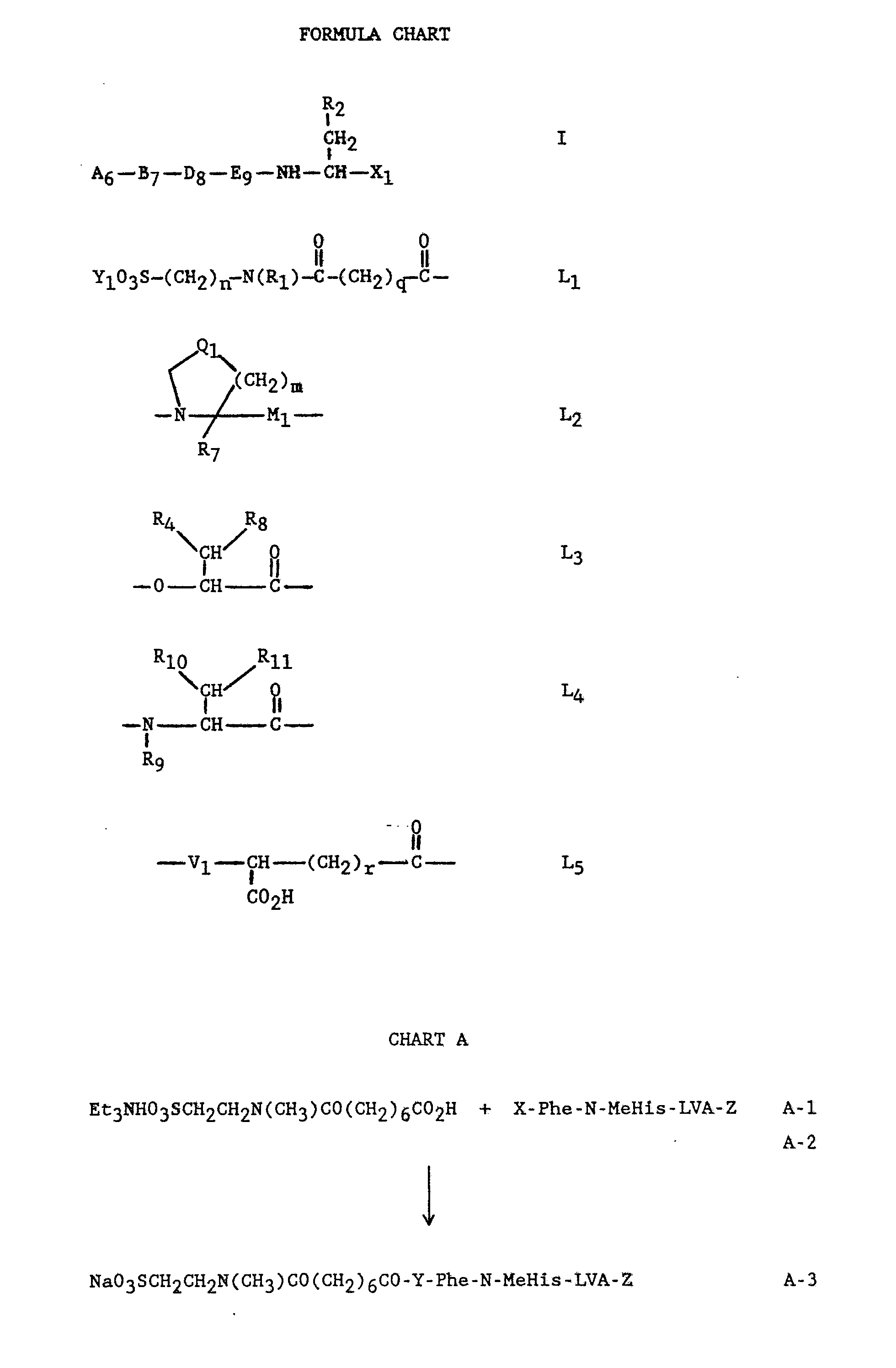

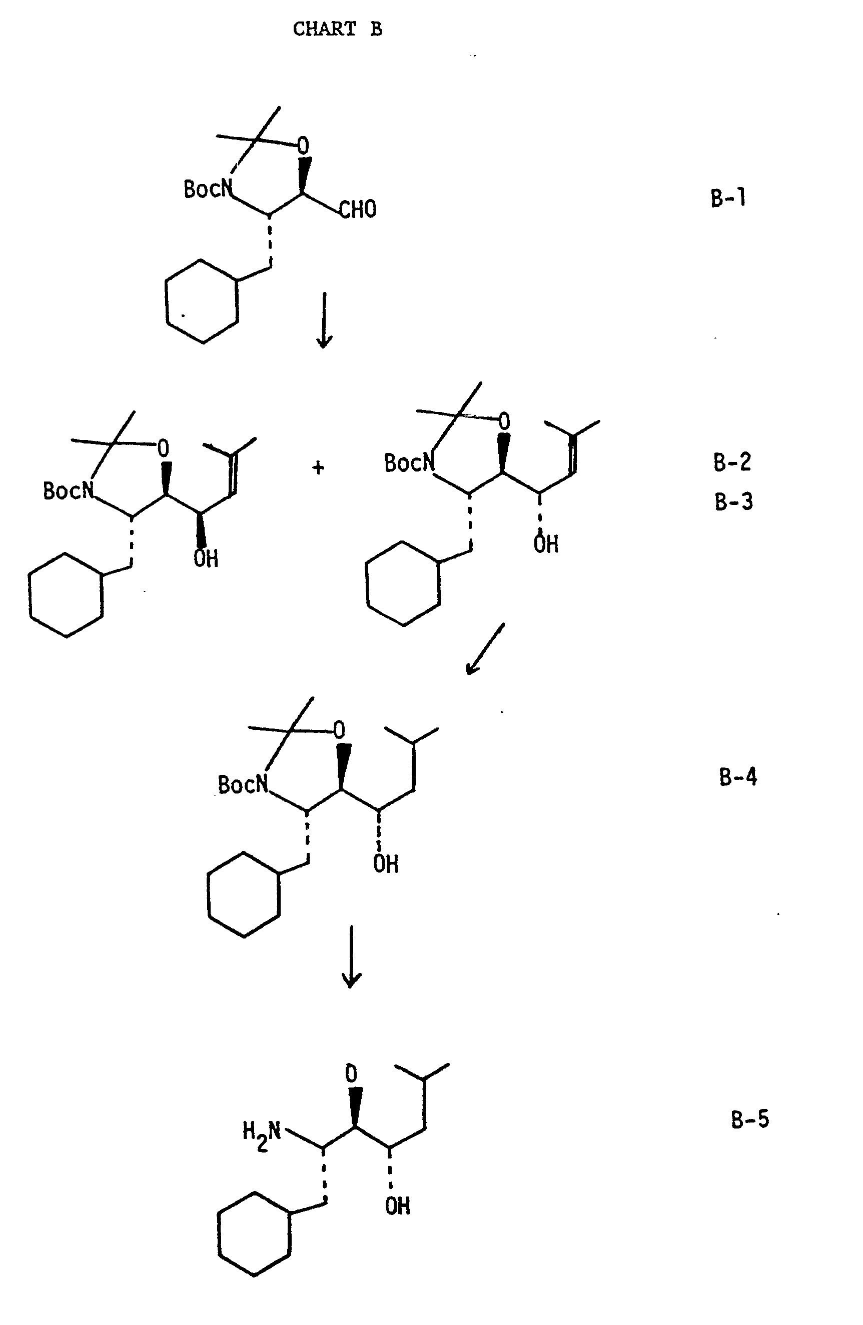

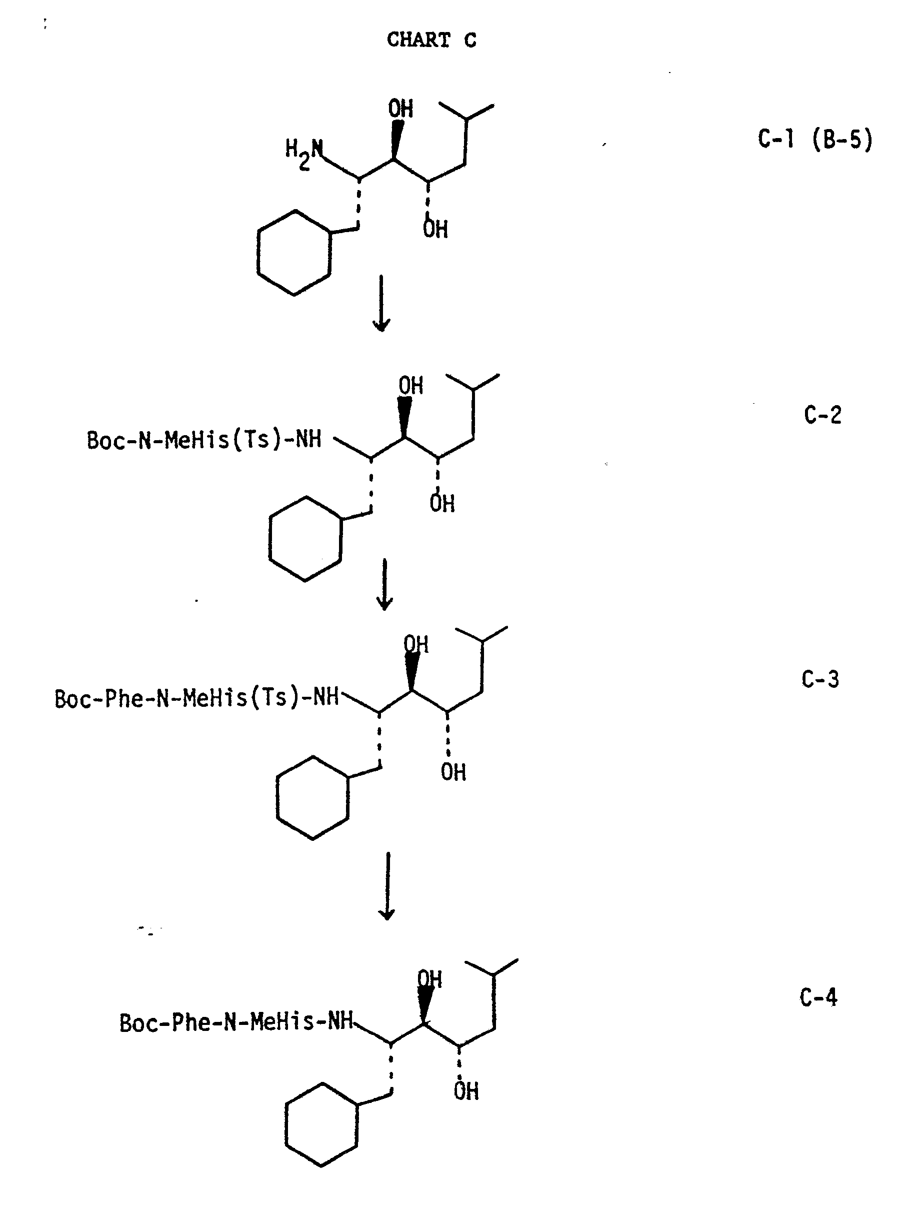

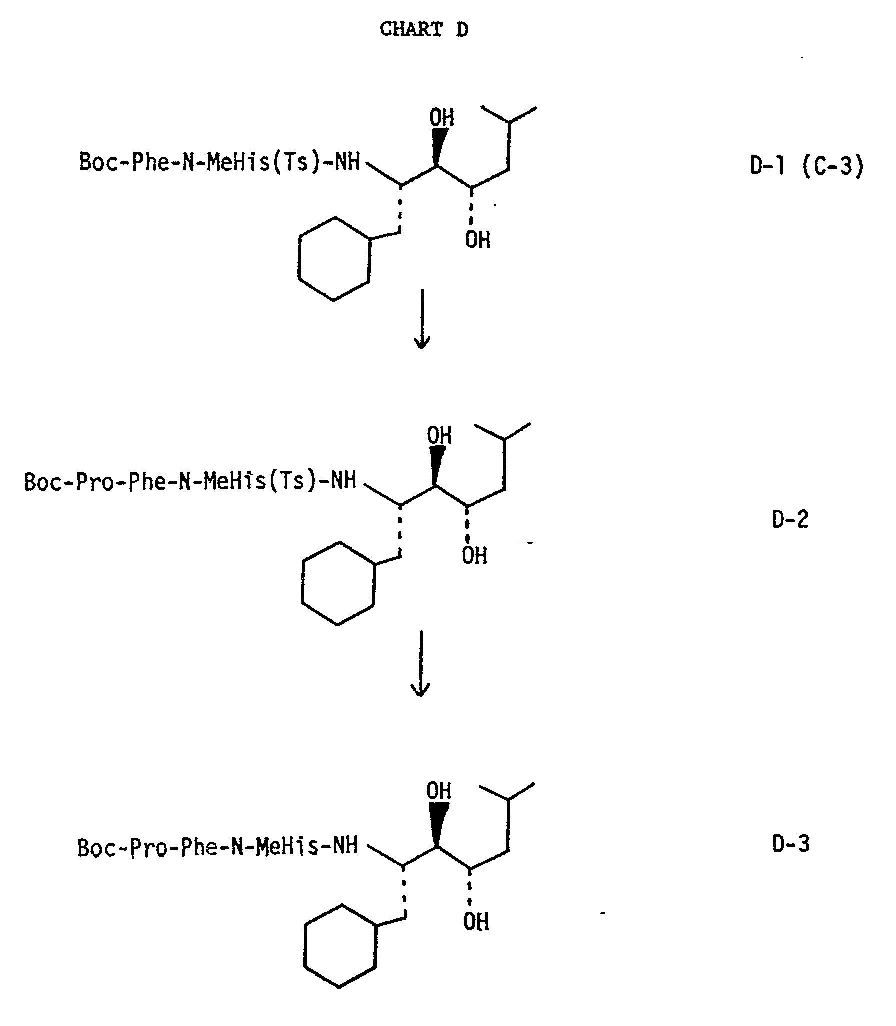

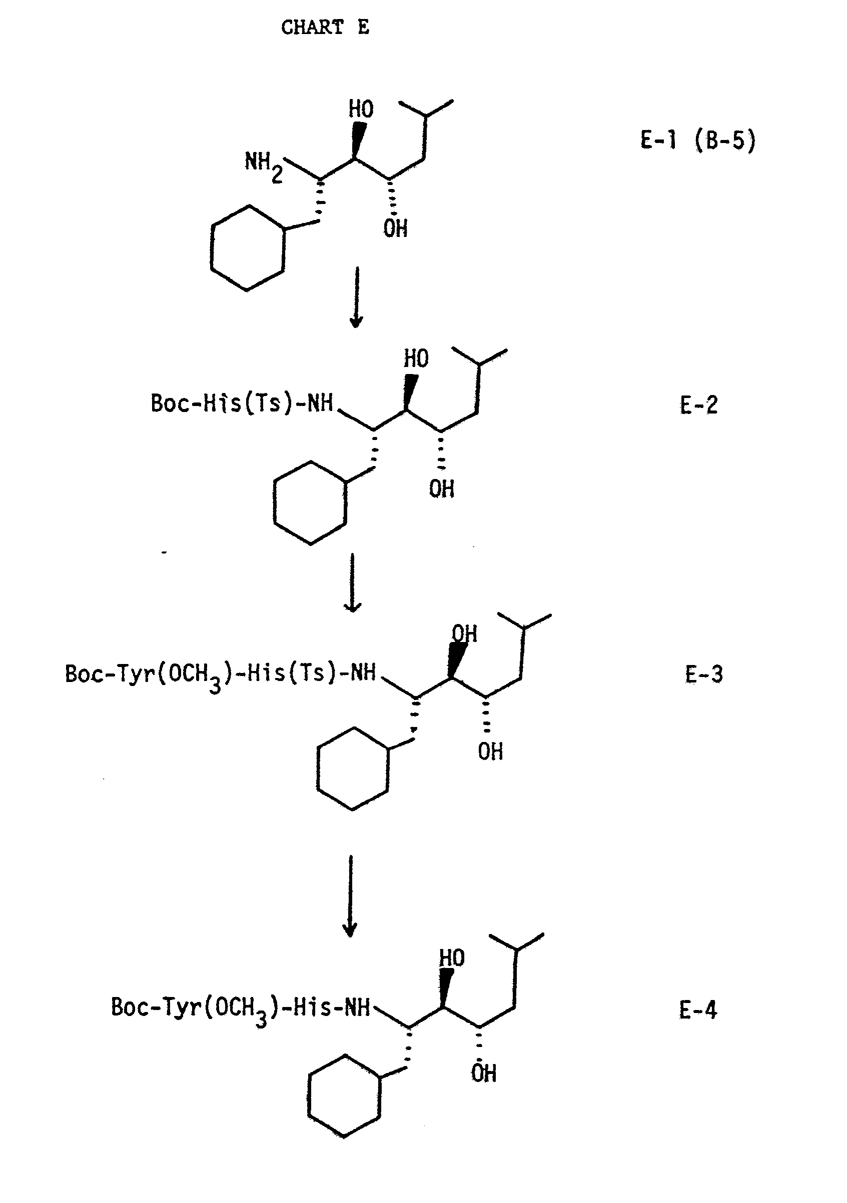

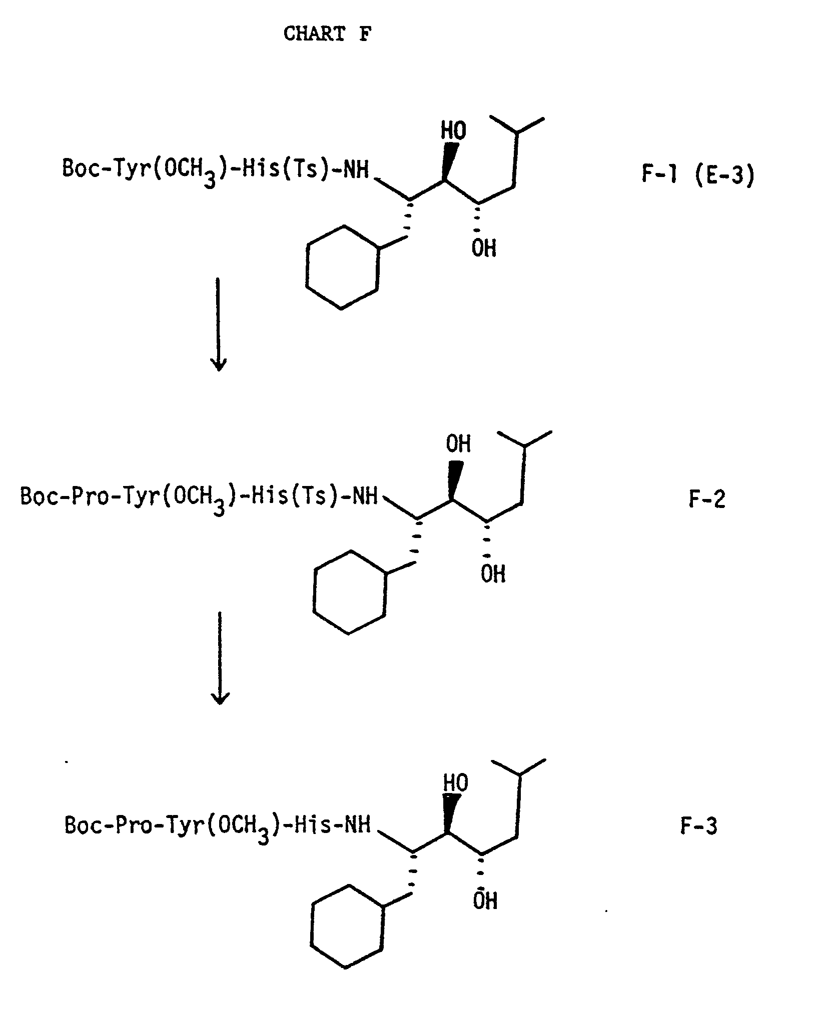

## Claims

1. A renin inhibitory peptide having a non-cleavable transition state insert corresponding to the 10,11-position of a renin substrate (angiotensinogen) and having a moiety of the formula L₁
Y₁0₃S-(CH₂)ₙ-N(R₁)- 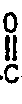 -(CH₂)_{q}- 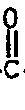 - L₁
at the N-terminus;
wherein Y₁ is
(a) hydrogen,
(b) alkali metals,
(c) alkali earth metals, or
(d) pharmaceutically acceptable base addition salts thereof;
wherein R₁ is
(a) hydrogen, or
(b) C₁-C₅alkyl;
wherein n is 1 to 5, inclusive; and
wherein q is 1 to 8, inclusive.

2. In a renin inhibitory peptide having a non-cleavable transition state insert corresponding to the 10,11-position of a renin substrate (angiotensinogen), the improvement which comprises inclusion in the renin inhibitory peptide of a moiety of the formula L₁
Y₁0₃S-(CH₂)ₙ-N(R₁)- 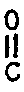 -(CH₂)_{q}- 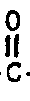 - L₁
at the N-terminus;
wherein Y₁ is
(a) hydrogen,
(b) alkali metals,
(c) alkali earth metals, or
(d) pharmaceutically acceptable base addition salts thereof;
wherein R₁ is
(a) hydrogen, or
(b) C₁-C₅alkyl;
wherein n is 1 to 5, inclusive; and
wherein q is 1 to 8, inclusive.

3. The renin inhibitory peptide of claim 1 of the formula I 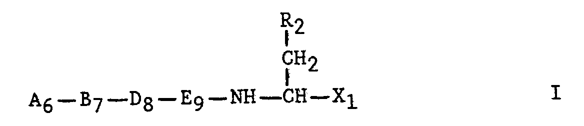 wherein A₆ is a monovalent moiety of the formula L₁;
Y₁O₃S-(CH₂)ₙ-N(R₁)- 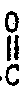 -(CH₂)_{q}- 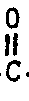 - L₁
wherein B₇ is absent or a divalent moiety of the formula L₂; 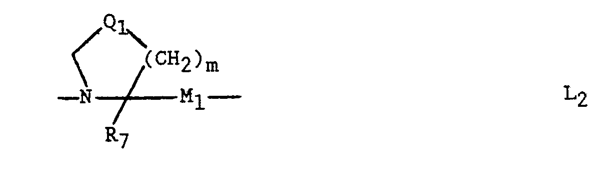 wherein D₈ is a divalent moiety of the formula L₃ or L₄; 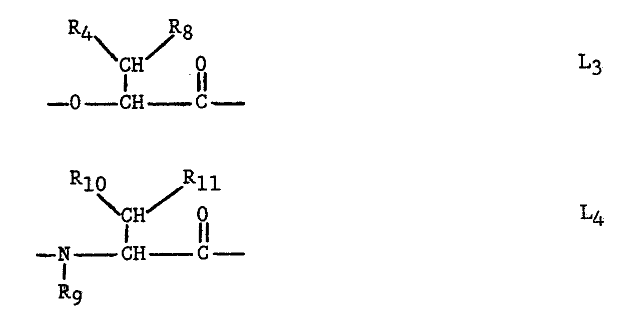 wherein E₉ is a divalent moiety of the formula L₄ or L₅; 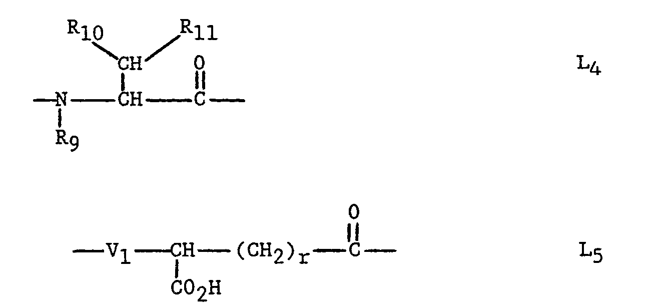 wherein each occurrence of the moiety of the formula L₄ can be the same or different;
wherein V₁ is
(a) -0-, or
(b) -N(R₁)-;
wherein X₁ is
(a) -CH(0H)-CH(0H)-CH₂-P₁,
(b) -L₁₀-C(R₁)(R₄)-C(0)-F₁-Z₁, or
(c) -J₁-C(K₁)(K₂)-C(0)-F₁-Z₁;
wherein P₁ is
(a) -N₃,
(b) -CN,
(c) C₁-C₆alkyl,
(d) C₁-C₆cycloalkyl,
(e) aryl, or
(f) Het;
wherein L₁₀ is a divalent moiety of the formula
(a) -CH(0H)-,
(b) -CH(NH₂)-,
(c) -C(0)-,
(d) -CH(0H)-C(K₁)(K₂)-,
(e) -C(0)-C(K₁)(K₂)-,
(f) -CH(0H)-CH(0H)-,
(g) -CH(0H)-CH₂-,
(h) -CH(NH₂)-CH₂-,
(i) -C(0)-CH₂-,
(j) -CH₂-NH-,
(k) -CH₂-0-, or
(l) -P₁(0)(G₁)-I₁-;
wherein R₁ is absent or a divalent moiety of the formula L₄; 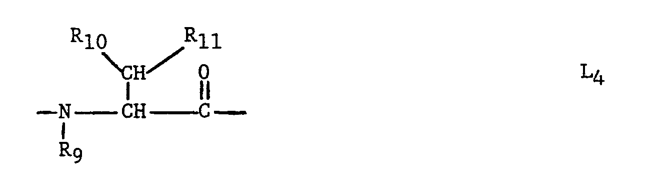 wherein G₁ is
(a) -0H, or
(b) -NH₂;
wherein I₁ is
(a) -0-,
(b) -NH-, or
(c) -CH₂;
wherein J₁ is
(a) -CH(0H)-,
(b) -CH(NH₂)-, or
(c) -C(0)-;
wherein K₁ and K₂ are the same or different and are
(a) H,
(b) F, or
(c) Cl;
wherein Q₁ is
(a) -CH₂-,
(b) -CH(0H)-,
(c) -0-, or
(d) -S-;
wherein M₁ is
(a) -C(0)-, or
(b) -CH₂-;
wherein Y₁ is
(a) hydrogen,
(b) alkali metals,
(c) alkali earth metals, or
(d) pharmaceutically acceptable base addition salts thereof;
wherein Z₁ is
(a) -0-R₅, or
(b) -N(R₁)R₅;
wherein R₁ is
(a) hydrogen, or
(b) C₁-C₅alkyl;
wherein R₂ is
(a) hydrogen,
(b) C₁-C₅alkyl,
(c) C₃-C₇cycloalkyl,
(d) aryl,
(e) Het,
(f) -(CH₂)ₚ-0H, or
(g) -(CH₂)ₚ-NH₂;
wherein R₃ is
(a) C₁-C₅alkyl,
(b) C₃-C₇cycloalkyl,
(c) aryl, or
(d) Het;
wherein R₄ is
(a) hydrogen,
(b) C₁-C₈alkyl,
(c) -(CH₂)ₚ-aryl,
(d) -(CH₂)ₚ-Het,
(e) -(CH₂)ₚ-C0₂H,
(f) -(CH₂)ₚ-NH₂,
(g) -(CH₂)ₚ-CH(NH₂)(C0₂H),
(h) C₃-C₇cycloalkyl, or
(i) 1- or 2-adamantyl;
wherein R₅ is
(a) hydrogen,
(b) C₁-C₁₀alkyl,
(c) aryl,
(d) Het,
(e) -(CH₂)ₚ-(C₃-C₇cycloalkyl),
(f) -(CH₂)ₚ-CH(NH₂)(C0₂H), or
(g) -(CH₂)ₙ-R₆;
wherein R₆ is
(a) aryl,
(b) Het,
(c) hydroxy,
(d) amino,
(e) C₁-C₅alkyl substituted by 1 to 3 -0H groups,
(f) -C0₂H,
(g) guanidinyl,
(h) -S0₃H, or
(i) -S0₂NH₂;
wherein R₇ is
(a) hydrogen, or
(b) C₁-C₅alkyl;
wherein R₈ is
(a) hydrogen, or
(b) C₁-C₅alkyl;
wherein R₉ is
(a) hydrogen, or
(b) C₁-C₅alkyl;
wherein R₁₀ is
(a) hydrogen,
(b) C₁-C₈alkyl,
(c) -(CH₂)ₚ-aryl,
(d) -(CH₂)ₚ-Het,
(e) -(CH₂)ₚ-C0₂H,
(f) -(CH₂)ₚ-NH₂,
(g) -(CH₂)ₚ-CH(NH₂)(C0₂H),
(h) C₃-C₇cycloalkyl, or
(i) 1- or 2-adamantyl;
wherein R₁₁ is
(a) hydrogen, or
(b) C₁-C₅alkyl;
wherein m is 1 or 2;
wherein n is 1 to 5, inclusive;
wherein p is 0 to 5, inclusive;
wherein q is 1 to 8, inclusive;
wherein r is 1 or 2;
wherein aryl is phenyl or naphthyl, optionally substituted by zero to three of the following;
(a) C₁-C₅alkyl,
(b) hydroxy,
(c) hydroxy (C₁-C₅alkyl),
(d) halogen,
(e) amino,
(f) amino (C₁-C₅alkyl),
(g) -CH0,
(h) -C0₂H,
(i) -C0₂-(C₁-C₅alkyl),
(j) -C0NH₂,
(k) -CONH-(C₁-C₅alkyl),
(l) nitro,
(m) mercapto,
(n) mercapto (C₁-C₅alkyl),
(o) -S0₃H,
(p) -S0₂NH₂,
(q) -CN, or
(r) -0-C₁-C₅alkyl;
wherein Het is a 5 or 6-membered saturated or unsaturated ring containing from one to three heteroatoms selected from the group consisting of nitrogen, oxygen and sulfur; and including any bicyclic group in which any of the above heterocyclic rings is fused to a benzene ring or another heterocycle; and if chemically feasible, the nitrogen and sulfur atoms may be in the oxidized forms; and optionally substituted by zero to three of the following;
(a) C₁-C₅alkyl,
(b) hydroxy,
(c) hydroxy (C₁-C₅alkyl),
(d) halogen,
(e) amino,
(f) amino (C₁-C₅alkyl),
(g) -CH0,
(h) -C0₂H,
(i) -C0₂-(C₁-C₅alkyl),
(j) -C0NH₂,
(k) -CONH-(C₁-C₅alkyl),
(l) nitro,
(m) mercapto,
(n) mercapto (C₁-C₅alkyl),
(o) -S0₃H,
(p) -S0₂NH₂,
(q) -CN, or
(r) -0-C₁-C₅alkyl;
or a carboxy-, amino-, or other reactive group protected form thereof;
or a pharmaceutically acceptable acid or base addition salts thereof.

4. The renin inhibitory peptide of claim 3
wherein A₆ is the moiety of formula L₁ wherein Y₁ is an alkali metal, n is 2, R₁ is methyl, and q is 6;
wherein B₇ is absent or the moiety of formula L₂ wherein Q₁ is -CH₂-, m is 1, R₇ is H, and M₁ is -C(0)-;
wherein D₈ is the moiety of formula L₄ wherein R₉ is hydrogen, R₁₀ is -(CH₂)ₚ-aryl, and R₁₁ is hydrogen;
wherein E₉ is the moiety of formula L₄ wherein R₉ is hydrogen or methyl, R₁₀ is -(CH₂)ₚ-Het, and R₁₁ is hydrogen; or
wherein E₉ is the moiety of formula L₅ wherein V₁ is -NH-, and r is 1;
wherein R₂ is C₁-C₅alkyl or C₃-C₇cycloalkyl;
wherein X₁ is -CH(0H)-CH(0H)-CH₂-P₁ wherein P₁ is C₁-C₆alkyl; or
wherein X₁ is -L₁₀-C(R₁)(R₄)-C(0)-F₁-Z₁ wherein L₁₀ is -CH(0H)CH₂-, R₁ is hydrogen, R₄ is C₁-C₈alkyl, F₁ is absent or the moiety of formula L₄ wherein R₉ is hydrogen, R₁₀ is C₁-C₈alkyl, and R₁₁ is C₁-C₅alkyl; and Z₁ is N(R₁)R₅ wherein R₁ is hydrogen and R₅ is -(CH₂)ₙ-R₆ or C₁-C₁₀alkyl; or
wherein X₁ is -J₁-C(K₁)(K₂)-C(0)-F₁-Z₁ wherein J₁ is -CH(0H)- or -C(0)-, K₁ is F, K₂ is F, F₁ is the moiety of formula L₄ wherein R₉ is hydrogen, R₁₀ is C₁-C₈alkyl, and R₁₁ is C₁-C₅alkyl; and Z₁ is N(R₁)R₅ wherein R₁ is hydrogen and R₅ is -(CH₂)ₙ-R₆.

5. A compound of claim 4 selected from the group consisting of:
N,N′-N-Methyltaurine, L-phenylalanyl-N^{α}-methyl-L-histidyl-5S-amino-4S-hydroxy-2S-isopropyl-7-methyloctanoyl-L-isoleucyl-2-pyridylmethylamine, octanoic acid diamide, sodium salt;
N,N′-N-Methyltaurine, L-prolyl-L-phenylalanyl-N^{α}-methyl-L-histidyl-5S-amino-4S-hydroxy-2S-isopropyl-7-methyloctanoyl-L-isoleucyl-2-pyridylmethylamine, octanoic acid diamide, sodium salt;
N,N′-N-Methyltaurine, L-phenylalanyl-N^{α}-methyl-L-histidyl-5S-amino-4S-hydroxy-2S-isopropyl-7-methyloctanoyl-2S-methylbutylamine, octanoic acid diamide, sodium salt;
N,N′-N-Methyltaurine, L-phenylalanyl-β-L-aspartyl-5S-amino-4S-hydroxy-2S-isopropyl-7-methyloctanoyl-2S-methylbutylamine, octanoic acid diamide, disodium salt;
N,N′-N-Methyltaurine-L-phenylalanyl-β-L-aspartyl-5S-amino-4S-hydroxy-2S-isopropyl-7-methyloctanoyl-L-isoleucyl-2-pyridylmethylamine, octanoic acid diamide, sodium salt;
N,N′-N-Methyltaurine-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, octanoic acid diamide, sodium salt;
N,N′-N-Methyltaurine, L-0-methyltyrosyl-L-histidyl-2S-amino-1-cyclohexyl-3R,4S-dihydroxy-6-methylheptane, octanoic acid diamide, sodium salt;
N,N′-N-Methyltaurine, L-phenylalanyl-N^{α}-methyl-L-histidyl-2S-amino-1-cyclohexyl-3R,4S-dihydroxy-6-methylheptane, octanoic acid diamide, sodium salt;
N,N′-N-Methyltaurine, L-phenylalanyl-β-L-aspartyl-5S-amino-4S-hydroxy-2S-isopropyl-7-methyloctanoyl-L-isoleucyl-1-oxa-2-pyridylmethylamine, octanoic acid diamide, sodium salt;
N,N′-N-Methyltaurine, L-phenylalanyl-L-histidyl-4S-amino-5-cyclohexyl-2,2-difluoro-3R-hydroxy-pentanoyl-L-isoleucyl-2-pyridylmethylamine, octanoic acid diamide, sodium salt;
N,N′-N-Methyltaurine, L-phenylalanyl-L-histidyl-4S-amino-5-cyclohexyl-2,2-difluoro-3-oxo-pentanoyl-L-isoleucyl-2-pyridylmethylamine, octanoic acid diamide, sodium salt;
N,N′-N-Methyltaurine, L-prolyl-L-phenylalanyl-N^{α}-methyl-L-histidyl-5S-amino-4S-hydroxy-2S-isopropyl-7-methyloctanoyl-L-isoleucyl-1-oxa-2-pyridylmethylamine, octanoic acid diamide, sodium salt;
N,N′-N-Methyltaurine, L-prolyl-L-0-methyltyrosyl-L-histidyl-2S-amino-1-cyclohexyl-3R,4S-dihydroxy-6-methylheptane, octanoic acid diamide, sodium salt;
N,N′-N-Methyltaurine, L-prolyl-L-phenylalanyl-N^{α}-methyl-L-histidyl-2S-amino-1-cyclohexyl-3R,4S-dihydroxy-6-methylheptane, octanoic acid diamide, sodium salt;
N,N′-N-Methyltaurine, L-prolyl-L-phenylalanyl-β-L-aspartyl-5S-amino-4S-hydroxy-2S-isopropyl-7-methyloctanoyl-L-isoleucyl-2-pyridylmethylamine, octanoic acid diamide, sodium salt; and
N,N′-N-Methyltaurine, L-prolyl-L-phenylalanyl-β-L-aspartyl-5S-amino-4S-hydroxy-2S-isopropyl-7-methyloctanoyl-L-isoleucyl-1-oxa-2-pyridylmethylamine, octanoic acid diamide, sodium salt.
